# FASCICULE DE BREVET EUROPEEN

(11) **EP 2 130 043 B1**
(45) Date de publication et mention de la délivrance du brevet: **07.12.2016**
(21) Numéro de dépôt: 08775669.8
(22) Date de dépôt: 04.03.2008
(51) Int. Cl.: G01N 33/497, C12Q 1/04

(54) **PROCÉDÉ DE DÉTECTION D'UNE CONTAMINATION FONGIQUE**
VERFAHREN ZUR ERKENNUNG EINER PILZKONTAMINATION
METHOD FOR DETECTING FUNGAL CONTAMINATION

(30) Priorité: 05.03.2007 FR 0701578
(43) Date de publication de la demande: 09.12.2009
(73) Titulaire: Centre Scientifique Et Technique Du Batiment (CSTB), 77420 Champs sur Marne (FR)
(72) Inventeur: MOULARAT, Stéphane, F-77185 Lognes (FR)
(74) Mandataire: Cabinet Plasseraud
(86) Numéro de dépôt international: PCT/FR2008/050366
(87) Numéro de publication internationale: WO 2008/125770

(56) Documents cités:
- SCHLEIBINGER H ET AL: "Emission patterns and emission rates of MVOC and the possibility for predicting hidden mold damage?" INDOOR AIR 2005, vol. 15 Suppl 9, 2005, pages 98-104, XP002457026 ISSN: 0905-6947
- CLAESON ANNA-SARA ET AL: "Volatile metabolites from microorganisms grown on humid building materials and synthetic media." JOURNAL OF ENVIRONMENTAL MONITORING : JEM OCT 2002, vol. 4, no. 5, octobre 2002 (2002-10), pages 667-672, XP002457027 ISSN: 1464-0325
- WALINDER ROBERT; WIESLANDER GUNILLA; NORBACK DAN; WESSEN BENGT; VENGE PER: "Nasal lavage biomarkers: Effects of water damage and microbial growth in an office building" ARCHIVES OF ENVIRONMENTAL HEALTH, vol. 56, 2001, pages 30-36, XP009091580
- GAO PENGFEI ET AL: "Determination of unique microbial volatile organic compounds produced by five Aspergillus species commonly found in problem buildings" AIHAJ, AMERICAN INDUSTRIAL HYGIENE ASSOCIATION, FAIRFAX, VA, US, vol. 63, no. 2, mars 2002 (2002-03), pages 135-140, XP009091499 ISSN: 1542-8117
- SCHLEIBINGER H; LAUSSMANN D; EIS D; SAMWER H; RUDEN H: "Sind MVOC geeignete Indikatoren für einen verdeckten Schimmelpilzbefall?" UMWELTMEDIZIN IN FORSCHUNG UND PRAXIS, vol. 9, 2004, pages 151-162, XP009091599

## Description

La présente invention concerne un procédé de détection d'une contamination fongique dans des environnements intérieurs.

Par environnement intérieur on entend un espace confiné à l'intérieur d'un bâtiment qui est aéré de façon non continu. Des exemples d'environnements intérieurs peuvent être trouvés dans les habitations, les musées, les églises, les caves, les monuments historiques, les bâtiments administratifs, les écoles et les hôpitaux.

Depuis les années 70 et le premier choc pétrolier, la politique d'économie d'énergie mise en oeuvre a entraîné un confinement des habitations avec notamment l'isolation accrue des bâtiments. Cette politique associée à la généralisation d'équipements ménagers générateurs de vapeur, tels que des lave-linge et des sèche-linge, a eu pour conséquence une augmentation de l'humidité relative favorable à la prolifération des microorganismes, notamment des moisissures, sur la plupart des supports tels que des matériaux de construction.

Les locaux sont alors susceptibles de constituer des « niches écologiques » pour le développement de ce type de microorganismes. Ce phénomène s'est ainsi traduit par une augmentation du nombre de locaux contaminés par les moisissures depuis les trente dernières années.

La présence de moisissures dans les environnements intérieurs n'est pas sans conséquences sanitaires. En effet, de nombreuses études ont démontré leur rôle à la fois dans la dégradation des matériaux et des ouvrages qu'elles colonisent, mais également dans l'apparition de symptômes chez les occupants de locaux comportant des moisissures.

Au cours des deux dernières décennies, de nombreuses études réalisées en Amérique du Nord et en Europe ont mis en évidence que, dans certaines circonstances d'exposition, ces microorganismes pouvaient être responsables de l'apparition de maladies notamment respiratoires telles que des allergies, infections ou toxi-infections.

Schleibinger H. et al., Umweltmed Forsch Prax 9(3) 151-162 (2004) décrit une étude visant à déterminer si les COV d'origine fongique peuvent être utilisés pour la détection de contamination fongique cachée. Pour cela, les concentrations de COV d'origine fongique ont été mesurées dans divers appartements et l'influence de divers facteurs, dont la présence de contamination fongique, sur les concentrations de ces COV a été déterminée.

A ce jour, les techniques utilisées pour détecter la présence de moisissures dans les environnements intérieurs reposent sur la reconnaissance visuelle d'un développement fongique et la mise en culture de conidies prélevées dans l'air ou sur les surfaces. Or, la présence de ces microorganismes peut se révéler difficile à diagnostiquer dans le cas de contaminations « masquées » pour lesquelles les spores ne sont pas émises dans l'environnement (lorsque les contaminations se situent au niveau du système de ventilation ou encore dans les structures du bâtiment par exemple). Invisibles alors à la surface des produits de construction, et indécelables par l'analyse microbiologique de l'air, les moisissures produisent néanmoins, en continu, des métabolites et produits de dégradation inhalables et responsables dans certains cas de maladies.

De plus, la réponse de ces techniques de mesure est longue puisqu'il est nécessaire d'attendre la croissance en laboratoire des microorganismes prélevés avant de pouvoir réaliser l'analyse.

Un objet de la présente invention est donc de pallier à tout ou partie des inconvénients cités ci-dessus.

Les champignons émettent dès le début de leur développement des molécules volatiles (Composés Organiques Volatils) issues soit de leur métabolisme, soit de la dégradation du matériau sur lequel ils se développent par les enzymes ou les acides qu'ils produisent. Contrairement aux spores, ces composés se dispersent dans l'environnement sans être retenus par les supports. Par conséquent la détection de certains de ces composés qui sont spécifiques d'une ou de plusieurs espèces fongiques, permet d'une part l'identification d'une contamination dès le début du développement des champignons et d'autre part la détection des contaminations « masquées » pour lesquelles les spores ne sont pas émises dans l'environnement.

La Société Demanderesse a maintenant trouvé que certains COV n'étaient présents dans l'air ambiant qu'en présence de moisissures. Ces COV sont donc spécifiquement issus du métabolisme fongique. La Société Demanderesse a également trouvé que certains autres COV étaient présents dans l'air ambiant non seulement en présence de moisissures, mais aussi en présence de certains matériaux de construction ou encore d'autres contaminations biologiques telles que les contaminations bactériennes. Un exemple d'un telle contamination bactérienne est la géosmine qui est émise à la fois par des moisissures et des bactéries. Forte de cette découverte, la Société Demanderesse a pu mettre en évidence que ces COV spécifiquement issus du métabolisme fongique peuvent être divisés en deux groupes :
- les COV qui sont émis indépendamment de l'espèce fongique et du support sur lequel se développe l'espèce fongique, et
- les COV qui sont émis en fonction de l'espèce fongique et/ou du support sur lequel elle se développe.

Parmi les COV qui sont émis indépendamment de l'espèce fongique et de son support, on distingue les COV qui ne sont émis que par des espèces fongiques et les COV qui peuvent avoir d'autres origines biologiques.

Elle a ainsi déterminé trois catégories distinctes de COV fongiques. La détection de ces COV est à la base de l'invention. C'est ainsi que la Société Demanderesse a eu le mérite, après des travaux de recherche longs et approfondis, de développer un procédé de détection d'une contamination fongique dans des environnements intérieurs qui permet la détection d'une telle contamination fongique même en l'absence de signes de contaminations visibles.

Ainsi, le procédé de détection d'une contamination fongique d'un environnement intérieur défini par un espace confiné à l'intérieur d'un bâtiment qui est aéré de façon non continu selon l'invention comprend les étapes suivantes :
a. prélèvement d'un échantillon de composés organiques volatils (COV) dans un environnement intérieur,
b. détection de la présence ou de l'absence de certains COV prédéterminés, issus du métabolisme fongique, ces COV prédéterminés comprenant au moins un COV de chacune des trois catégories de COV suivantes :
   (1) COV qui sont émis indépendamment de l'espèce fongique et de leur support et qui ne sont émis que par des espèces fongiques ;
   (2) COV qui sont émis indépendamment de l'espèce fongique et du support, mais qui peuvent également avoir d'autres origines biologiques ; et
   (3) COV qui sont émis en fonction de l'espèce fongique et/ou du support ;
c. calcul d'un indice chimique de contamination fongique en fonction respectivement de la présence et de l'absence des COV prédéfinis, issus du métabolisme fongique, en prenant en considération que:
   la présence de COV de catégorie (1) indique directement la présence d'une contamination fongique tandis que l'absence de tels COV indique l'absence d'une contamination fongique ;
   la présence de COV de catégorie (2) ne permet pas de conclure sur une contamination fongique tandis que l'absence de tels COV indique l'absence d'une contamination fongique; et
   la présence de COV de catégorie (3) indique la présence d'une contamination fongique tandis que leur absence ne permet pas de conclure sur l'absence d'une contamination fongique.

Les COV qui ne sont émis que par des espèces fongiques et dont l'émission est indépendante du support comprennent notamment le 1-octèn-3-ol, le 1,3-octadiène et le méthyl-2-éthylhexanoate. Les COV qui peuvent également avoir d'autres origines biologiques dont l'émission est indépendante du support comprennent le 2-méthylfurane, le 3-méthylfurane, le 3-méthyl-1-butanol, le 2- méthyl-1-butanol et le α-pinène. Les COV qui sont émis en fonction de l'espèce fongique et/ou du support comprennent notamment le 2-heptène, le diméthylsulfide, le 4-heptanone, le 2(5H)-furanone, le 3-heptanol et le méthoxybenzène.

Dans un mode de réalisation, les COV prédéterminés comprennent outre les COV des catégories (1), (2), (3) également le 2-éthylhexanol.

Conformément au procédé de l'invention, l'étape a) de prélèvement de l'échantillon de COV est de préférence réalisée par échantillonnage diffusif sur un adsorbant solide de type carbographe 4.

A partir de l'échantillon de COV prélevé à l'étape a) on détecte la présence ou l'absence de certains COV prédéterminés, issus du métabolisme fongique. Ces COV prédéterminés comprennent au moins
- un COV qui est émis indépendamment de l'espèce fongique et de son support et qui n'est émis que par des espèces fongiques ;
- un COV qui est émis indépendamment de l'espèce fongique et du support, mais qui peut également avoir d'autres origines biologiques ; et
- un COV qui sont émis en fonction de l'espèce fongique et/ou du support.

En détectant la présence ou l'absence d'au moins un COV de chacune des trois catégories de COV citées ci-dessus, on augmente la certitude du procédé de détection selon l'invention par rapport à une détection de la présence ou l'absence de COV issus d'une seule de ces catégories.

De préférence, on détectera plusieurs COV de chacune des trois catégories pré-citées.

Dans un mode de réalisation avantageux, les COV de la catégorie (1) sont choisis dans le groupe comprenant le 1-octen-3-ol, le 1,3-octadiène et le méthyl-2-éthylhexanoate, les COV de la catégorie (2) sont choisis dans le groupe comprenant le 2-méthylfurane, le 3-méthylfurane, le 3-méthyl-1-butanol, le 2- méthyl-1-butanol et le α-pinène, et les COV de la catégorie (3) sont choisis dans le groupe comprenant le 2-heptène, le diméthylsulfide, le 4-heptanone, le 2(5H)-furanone et le 3-heptanol.

De préférence, les COV prédéterminés sont détectés par chromatographie en phase gazeuse suivie de spectrométrie de masse (GC/MS).

Après la détection de la présence ou l'absence de chacun des COV prédéterminés, on calcule un indice chimique de contamination fongique. Ce calcul est basé sur les constatations suivantes, que la Société Demanderesse a pu faire après des recherches longues et approfondies.

La présence de COV spécifiquement issus du métabolisme fongique, qui sont émis indépendamment de l'espèce fongique et de son support et qui ne sont émis que par des espèces fongiques, indique directement la présence d'une contamination fongique tandis que l'absence de tels COV indique l'absence d'une contamination fongique.

Par contre, la présence de COV issus du métabolisme fongique, qui peuvent aussi avoir d'autres origines biologiques ne permet pas de conclure sur une contamination fongique. Toutefois, l'absence de tels COV indique l'absence d'une contamination fongique.

En ce qui concerne les COV spécifiquement issus du métabolisme fongique mais qui sont émis en fonction de l'espèce fongique et/ou du support, leur présence indique la présence d'une contamination fongique, tandis que leur absence ne permet pas de conclure sur l'absence d'une contamination fongique.

Un cas particulier est le méthyl-2-éthylhexanoate qui, à la base, fait partie des COV émis indépendamment de l'espèce fongique et de son support, mais dont la formation semble être due à la transformation de 2-éthylhexanol par la moisissure. Sans vouloir être lié par une quelconque théorie, il est supposé que le 2-éthylhexanol se transforme en méthyl-2-éthylhexanoate par l'intermédiaire d'une réaction d'oxydation en acide 2-éthylhexanoïque lequel est ensuite estérifié en méthyl-2-éthylhexanoate. La présence de méthyl-2-éthylhexanoate permet de conclure à la présence d'une contamination fongique. En revanche, en cas d'absence de méthyl-2-éthylhexanoate, il est important de détecter la présence ou l'absence de 2-éthylhexanol. Si, dans ce cas, le 2-éthylhexanol est présent, on peut conclure à l'absence d'une contamination fongique. Si, par contre, le 2-éthylhexanol est absent, on ne peut pas conclure à la présence d'une contamination fongique.

Sur la base de ces constatations, la Société Demanderesse a eu le mérite de mettre au point une méthode de calcul d'un indice chimique de contamination fongique qui est basé sur l'incrémentation suivante. La présence d'un COV est incrémentée d'une valeur « 1 » si la présence du COV indique la présence d'une contamination fongique et d'une valeur « 0 » si la présence du COV ne permet pas de conclure à la présence d'une contamination fongique. L'absence d'un COV est incrémentée d'une valeur « -1 » si l'absence du COV indique l'absence d'une contamination fongique et d'une valeur « 0 » si l'absence du COV ne permet pas de conclure à l'absence d'une contamination fongique. Le tableau 1 ci-dessous résume les principes d'incrémentation.

**Tableau 1: Incrémentation**

| | Incrémentation | |
|---|---|---|
| COV | présence | absence |
| émis indépendamment de l'espèce fongique et de son support, émis que par des espèces fongiques | 1 | -1 |
| émis indépendamment de l'espèce fongique et du support, peut également avoir d'autres origines biologiques | 0 | -1 |
| émis en fonction de l'espèce fongique et/ou du support | 1 | 0 |

Dans le cas particulier du méthyl-2-éthylhexanoate la présence du méthyl-2-hexanoate est incrémentée d'une valeur « 1 », tandis que son absence est incrémentée d'une valeur « -1 » si le 2-éthylhexanol est présent dans l'échantillon et d'une valeur « 0 » si le 2-éthylhexanol est absent de l'échantillon. Le tableau 2 résume le principe d'incrémentation par rapport au méthyl-2-hexanoate.

**Tableau 2: Incrémentation méthyl-2-éthylhexanoate**

| COV | Incrémentation | | |
|---|---|---|---|
| | présence | absence | |
| | | 2-éthylhexanol présent | 2-éthylhexanol absent |
| méthyl-2-hexanoate | 1 | -1 | 0 |

L'indice chimique de contamination fongique est calculé par addition des incréments qui ont été attribués à la présence ou à l'absence de chacun des COV prédéterminés. Le résultat de cette addition, c'est-à-dire l'indice chimique de contamination fongique est donc soit une valeur négative, soit égale à zéro, soit une valeur positive. Si l'indice chimique de contamination fongique est une valeur inférieure ou égale à 0, ceci indique l'absence d'une contamination fongique. Un indice de contamination fongique strictement positif indique la présence d'une contamination fongique.

Le procédé de détection d'une contamination fongique selon l'invention est particulièrement utile pour la détection précoce d'une telle contamination, c'est-à-dire avant l'apparition de signes visibles de contamination. Cette possibilité de détection précoce est par exemple de grande importance dans des monuments historiques, des églises ou des musées, où des dommages irréparables ont en général déjà été causés quand les premiers signes visibles d'une contamination fongique apparaissent.

Les exemples de réalisation suivants illustrent la présente invention, sans en limiter en aucune façon la portée.

### EXEMPLE 1 : Prélèvement d'un échantillon de COV dans un environnement intérieur

Des prélèvements de COV *in situ* ont été réalisés par échantillonnage diffusif sur un adsorbant solide de type carbograph 4 dans douze logements. Cinq des douze logements contenaient au moins une pièce ayant une tache de moisissure visible supérieure à 1 m² (habitations 1 à 5) et sept des douze logements ne présentaient pas de signes visibles de contamination fongique (habitations 7 à 12). Le prélèvement est assuré par un tube à diffusion. L'échantillonneur est composé d'une cartouche, d'un corps diffusif et d'un adaptateur.

La cartouche est cylindrique (40-60 mesh s.s. net), de diamètre externe 4,8 mm, et contenant 300 mg de charbon graphitisé (Carbograph 4). Cette cartouche est placée avant le prélèvement dans un corps diffusif en polyéthylène. L'ensemble est ensuite vissé sur un portoir à l'aide d'un support clipable.

Les tubes passifs sont exposés sur site pendant une durée de 7 jours. Le point de prélèvement se situe entre 0,5 et 1 m de hauteur. Après exposition, les cartouches sont conservées au réfrigérateur avant analyse.

La majeur partie des COV (fongique ou non) composant l'air du logement sont alors piégés dans l'adsorbant.

### EXEMPLE 2 : Détection de la présence ou l'absence de certains COV

Les tubes contenant l'adsorbant sont transférés dans une chaîne analytique du laboratoire. Cette chaîne consiste en la combinaison de trois techniques :
- la chromatographie en phase gazeuse (GC) utilisée pour séparer les COV,
- l'ionisation de flamme (FID) qui permet la détection des diverses molécules,
- la spectrométrie de masse (MS) employée pour identifier ces composés.

Pour chacun des douze logements, on obtient donc des chromatogrammes et les COV spécifiques y sont recherchés (voir tableau I pour les COV recherchés).

### EXEMPLE 3 : Calcul de l'indice chimique de contamination fongique

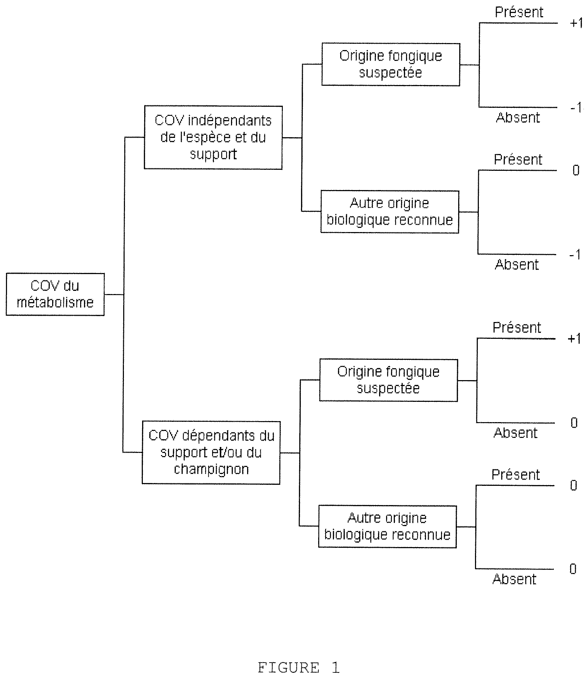

Un indice de contamination est ensuite calculé afin de regrouper l'ensemble des informations fournies par la présence ou l'absence des COV spécifiques identifiés. Afin de formaliser l'ensemble des observations faite sur l'origine de ces molécules, l'indice est calculé en fonction de la présence ou de l'absence de chacun de ces COV. La méthode d'incrémentation de cet indice est schématisée sur la figure 1.

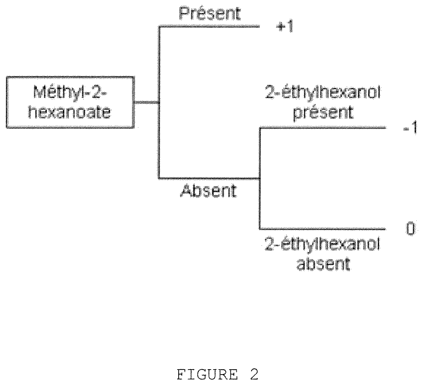

Concernant le méthyl-2-éthylhexanoate, nous avons pris en compte que sa formation pourrait être due à la transformation de 2-éthylhexanol par la moisissure. La règle d'incrémentation que nous avons appliquée dans ce cas est décrite dans la figure 2.

D'après la construction de cet indice, une valeur élevée rend probable la présence d'un développement fongique, a contrario, une valeur faible l'exclut.

Ainsi, une valeur d'indice strictement supérieur à 0 permet de conclure sur la présence d'un développement fongique dans le logement étudié alors qu'une valeur négative ou nulle l'exclu.

Les résultats de l'analyse des chromatogrammes de prélèvements réalisés dans les 12 logements sont référencés dans le tableau I.

**TABLEAU I : Liste des COV issus du métabolisme identifiés dans 12 habitations (0 = absence du composé, 1 = présence du composé)**

| **Composés** | **Habitation avec contamination visible** | | | | | **Habitation sans contamination visible** | | | | | | |
|---|---|---|---|---|---|---|---|---|---|---|---|---|
| | **Hab. 1** | **Hab. 2** | **Hab. 3** | **Hab. 4** | **Hab. 5** | **Hab. 6** | **Hab. 7** | **Hab. 8** | **Hab. 9** | **Hab. 10** | **Hab. 11** | **Hab. 12** |
| 1-octen-3-ol | 0 | 0 | 0 | 0 | 1 | 0 | 0 | 0 | 0 | 0 | 0 | 0 |
| 1,3-octadiène | 1 | 1 | 1 | 1 | 1 | 1 | 0 | 0 | 1 | 0 | 0 | 0 |
| méthyl-2-éthylhexanoate | 0 | 0 | 0 | 0 | 0 | 0 | 0 | 0 | 0 | 0 | 0 | 0 |
| 2-éthylhexanol | 1 | 1 | 1 | 1 | 1 | 1 | 1 | 1 | 1 | 1 | 1 | 1 |
| α-pinène | 1 | 1 | 1 | 1 | 1 | 1 | 1 | 1 | 1 | 1 | 1 | 1 |
| 2-méthylfurane | 1 | 1 | 1 | 1 | 1 | 0 | 0 | 1 | 1 | 1 | 1 | 0 |
| 3-méthyl-1-butanol | 1 | 0 | 1 | 1 | 1 | 1 | 1 | 1 | 1 | 1 | 1 | 1 |
| 2-méthyl-1-butanol | 0 | 0 | 0 | 0 | 0 | 0 | 0 | 0 | 0 | 0 | 0 | 0 |
| Diméthyldisulfide | 1 | 1 | 1 | 0 | 0 | 0 | 0 | 0 | 1 | 0 | 0 | 0 |
| 2-héptène | 1 | 1 | 1 | 1 | 1 | 0 | 0 | 0 | 1 | 1 | 1 | 1 |
| 2-méthylisobornéol | 0 | 0 | 0 | 0 | 0 | 0 | 0 | 0 | 0 | 0 | 0 | 0 |
| sesquiterpène A | 0 | 0 | 0 | 0 | 0 | 0 | 0 | 0 | 0 | 0 | 0 | 0 |
| Sesquiterpène B | 1 | 1 | 0 | 1 | 1 | 0 | 1 | 0 | 1 | 0 | 0 | 0 |
| Sesquiterpène C | 0 | 0 | 0 | 0 | 0 | 0 | 0 | 0 | 0 | 0 | 0 | 0 |
| Terpénoïde | 0 | 0 | 0 | 0 | 0 | 0 | 0 | 0 | 0 | 0 | 0 | 0 |
| 4-heptanone | 1 | 1 | 1 | 0 | 0 | 0 | 0 | 0 | 0 | 0 | 0 | 0 |
| 3-heptanol | 0 | 0 | 0 | 0 | 0 | 0 | 0 | 0 | 0 | 0 | 0 | 0 |
| Méthoxybenzène | 0 | 0 | 0 | 0 | 0 | 0 | 0 | 0 | 0 | 0 | 0 | 0 |
| 2(5H)-furanone | 1 | 1 | 0 | 1 | 0 | 0 | 0 | 0 | 0 | 0 | 0 | 0 |

Nous constatons la présence systématique de l'α-pinène et du 3-méthyl-1-butanol. Cette observation confirme l'hypothèse que ces composés possèdent de nombreuses sources d'émission (moisissures, bactéries, plantes).

L'indice de contamination établi a ensuite été appliqué pour chaque habitation (voir Figures 1 et 2). Les valeurs de cet indice sont répertoriées dans le tableau II:

**Tableau II : Valeurs des indices attribuées à chaque habitation**

| **Habitation avec contamination visible** | **Valeur de l'indice** | **Habitation sans contamination visible** | **Valeur de l'indice** |
|---|---|---|---|
| H1 | 4 | H6 | -3 |
| H2 | 3 | H7 | -4 |
| H3 | 2 | H8 | -3 |
| H4 | 3 | H9 | 2 |
| H5 | 3 | H10 | 0 |
| | | H11 | 0 |
| | | H12 | -4 |

Les habitations présentant une contamination visible ont toutes un indicateur strictement positif. Cette valeur positive de l'indice confirme donc la présence probable d'un développement fongique dans ces habitations.

A l'exception de l'habitation n°9, les habitations sans contamination visible ont des indicateurs inférieurs ou égaux à 0. La valeur des indices est donc corrélée à la présence d'un développement fongique.

L'habitation n°9, classée dans les logements *a priori* « sains », se comporte, en termes de traceurs chimiques, de la même manière que les habitations dont la contamination fongique est avérée (indicateur strictement supérieur à 0). Ces habitations ne présentent donc pas de risque de contamination fongique.

Pour vérifier ces hypothèses, nous associons ces résultats à un questionnaire soumis aux occupants de chaque habitation. Parmi les différentes questions, six pouvaient être associées à la présence de nos traceurs. Ces questions ainsi que les réponses associées pour chaque habitation sont répertoriées dans le tableau III.

**Tableau III : Résultats du questionnaire soumis aux propriétaires des douze habitations (1=oui, 0=non)**

| | Moisissures visibles sur plus 1m² | Présence d'humidité dans l'habitation | Infiltrations d'eau au cours des 12 derniers mois | Traitement contre l'humidité | Présence de plantes | Odeur de moisi détecté par les enquêteurs |
|---|---|---|---|---|---|---|
| **Habitation 1** | 1 | 1 | 0 | 0 | 0 | 1 |
| **Habitation 2** | 1 | 1 | 1 | 0 | 1 | 1 |
| **Habitation 3** | 1 | 1 | 1 | 1 | 1 | 1 |
| **Habitation 4** | 1 | 1 | 1 | 0 | 1 | 0 |
| **Habitation 5** | 1 | 1 | 1 | 0 | 1 | 0 |
| **Habitation 6** | 0 | 0 | 0 | 0 | 1 | 0 |
| **Habitation 7** | 0 | 0 | 0 | 0 | 1 | 0 |
| **Habitation 8** | 0 | 0 | 0 | 0 | 1 | 0 |
| **Habitation 9** | 0 | 0 | 1 | 0 | 1 | 0 |
| **Habitation 10** | 0 | 0 | 0 | 0 | 0 | 0 |
| **Habitation 11** | 0 | 0 | 0 | 0 | 1 | 0 |
| **Habitation 12** | 0 | 0 | 0 | 0 | 1 | 0 |

Nous constatons que l'habitation n°9 (sans signe visible mais avec un indice positif) a subi des infiltrations d'eau au cours des 12 mois précédant le prélèvement. Il est probable que ces infiltrations aient engendré une contamination fongique qui n'a pas été détectée visuellement. Par conséquent, l'indice établi a permis de détecter une contamination avant l'apparition des premières traces visibles d'une croissance fongique.

## Revendications

1. Procédé de détection d'une contamination fongique d'un environnement intérieur défini par un espace confiné à l'intérieur d'un bâtiment qui est aéré de façon non continu comprenant les étapes suivantes :
a. prélèvement d'un échantillon de composés organiques volatils (COV) dans un environnement intérieur,
b. détection de la présence ou l'absence de certains COV prédéterminés, issus du métabolisme fongique, ces COV prédéterminés comprenant au moins un COV de chacune des trois catégories de COV suivantes :
(1) les COV qui sont émis indépendamment de l'espèce fongique et de son support et qui ne sont émis que par des espèces fongiques ;
(2) les COV qui sont émis indépendamment de l'espèce fongique et du support, mais qui peuvent également avoir d'autres origines biologiques ;
(3) les COV qui sont émis en fonction de l'espèce fongique et/ou du support ;
c. calcul d'un indice chimique de contamination fongique en fonction respectivement de la présence et de l'absence des COV prédéfinis, issus du métabolisme fongique, en prenant en considération que :
la présence de COV de catégorie (1) indique directement la présence d'une contamination fongique tandis que l'absence de tels COV indique l'absence d'une contamination fongique ;
la présence de COV de catégorie (2) ne permet pas de conclure sur une contamination fongique tandis que l'absence de tels COV indique l'absence d'une contamination fongique ; et
la présence de COV de catégorie (3) indique la présence d'une contamination fongique tandis que leur absence ne permet pas de conclure sur l'absence d'une contamination fongique.

2. Procédé de détection d'une contamination fongique selon la revendication 1, **caractérisé en ce que** l'on détecte la présence ou l'absence de plusieurs COV de chacune des trois catégories de COV.

3. Procédé de détection d'une contamination fongique selon la revendication 1 ou 2, **caractérisé en ce que** les COV prédéterminés comprennent outre les COV des catégories (1), (2), (3) également le 2-éthylhexanol.

4. Procédé de détection d'une contamination fongique selon l'une quelconque des revendications 1 à 3, **caractérisé en ce que**
- les COV de la catégorie (1) sont choisis dans le groupe comprenant le 1-octen-3-ol, le 1,3-octadiène et le méthyl-2-éthylhexanoate,
- les COV de la catégorie (2) sont choisis dans le groupe comprenant le 2-méthylfurane, le 3-méthylfurane, le 3-méthyl-1-butanol, le 2-méthyl-1-butanol et le α-pinène,
- les COV de la catégorie (3) sont choisis dans le groupe comprenant le 2-heptène, le diméthylsulfide, le 4-heptanone, le 2(5H)-furanone et le 3-heptanol.

5. Procédé de détection d'une contamination fongique selon l'une quelconque des revendications 1 à 4, **caractérisé en ce que** dans l'étape c), on attribue à chacun des COV prédéterminés un chiffre de -1, 0 ou 1 en fonction de sa présence ou de son absence et on calcule la somme de ces chiffres qui est l'indice de contamination.

6. Procédé de détection d'une contamination fongique selon la revendication 5, **caractérisé en ce que**, pour chacun des COV à l'exception du méthyl-2-éthylhexanoate, l'attribution des chiffres se fait de la façon suivante :
- la présence de COV de la catégorie (1) est **caractérisée par** le chiffre 1 et son absence par -1 ;
- la présence de COV de la catégorie (2) est **caractérisée par** le chiffre 0 et son absence par -1 ;
- la présence de COV de la catégorie (3) est **caractérisée par** le chiffre 1 et son absence par 0.

7. Procédé de détection d'une contamination fongique selon la revendication 5 ou 6, **caractérisé en ce que**, pour le méthyl-2-éthylhexanoate, l'attribution des chiffres se fait de la façon suivante :
- La présence de méthyl-2-hexanoate est **caractérisée par** le chiffre 1 ;
- L'absence de méthyl-2-éthylhexanoate est **caractérisée par** le chiffre 0, si on détecte une absence de 2-éthylhexanol, et par le chiffre -1, si on détecte un présence de 2-éthylhexanol.

## Patentansprüche

1. Verfahren zum Nachweis einer Pilzkontamination einer inneren Umgebung, definiert durch einen geschlossenen Raum im Inneren eines Gebäudes, der nicht auf kontinuierliche Weise belüftet wird, umfassend die folgenden Schritte:
a. Entnehmen einer Probe von flüchtigen organischen Verbindungen (VOC) in einer inneren Umgebung,
b. Nachweis des Vorliegens oder der Abwesenheit bestimmter vorgegebener VOC, die aus dem Pilzstoffwechsel stammen, wobei diese vorgegebenen VOC mindestens eine VOC aus jeder der drei folgenden VOC-Kategorien umfassen:
(1) die VOC, die unabhängig von der Pilzspezies und ihrem Träger emittiert werden und die nur von Pilzspezies emittiert werden;
(2) die VOC, die unabhängig von der Pilzspezies und dem Träger emittiert werden, aber die auch anderen biologischen Ursprung haben können;
(3) die VOC, die abhängig von der Pilzspezies und/oder dem Träger emittiert werden;
c. Berechnen eines chemischen Index der Pilzkontamination als Funktion des Vorliegens bzw. der Abwesenheit der vorgegebenen VOC, die aus dem Pilzstoffwechsel stammen, unter Berücksichtigung der Tatsache, dass:
das Vorliegen von VOC der Kategorie (1) direkt das Vorliegen einer Pilzkontamination anzeigt, wohingegen die Abwesenheit solcher VOC die Abwesenheit einer Pilzkontamination anzeigt;
das Vorliegen von VOC der Kategorie (2) es nicht erlaubt, Rückschlüsse auf eine Pilzkontamination zu ziehen, wohingegen die Abwesenheit solcher VOC die Abwesenheit einer Pilzkontamination anzeigt; und
das Vorliegen von VOC der Kategorie (3) das Vorliegen einer Pilzkontamination anzeigt, wohingegen ihre Abwesenheit es nicht erlaubt, Rückschlüsse auf die Abwesenheit einer Pilzkontamination zu ziehen.

2. Verfahren zum Nachweis einer Pilzkontamination gemäß Anspruch 1, **dadurch gekennzeichnet, dass** das Vorliegen oder die Abwesenheit von mehreren VOC jeder der drei Kategorien von VOC nachgewiesen wird.

3. Verfahren zum Nachweis einer Pilzkontamination gemäß Anspruch 1 oder 2, **dadurch gekennzeichnet, dass** die vorgegebenen VOC außer den VOC der Kategorien (1), (2) und (3) auch 2-Ethylhexanol umfassen.

4. Verfahren zum Nachweis einer Pilzkontamination gemäß einem der Ansprüche 1 bis 3, **dadurch gekennzeichnet, dass**
- die VOC der Kategorie (1) ausgewählt sind aus der Gruppe umfassend 1-Okten-3-ol, 1,3-Oktadien und Methyl-2-ethylhexanoat,
- die VOC der Kategorie (2) ausgewählt sind aus der Gruppe umfassend 2-Methylfuran, 3-Methylfuran, 3-Methyl-1-butanol, 2-Methyl-1-butanol und α-Pinen,
- die VOC der Kategorie (3) ausgewählt sind aus der Gruppe umfassend 2-Hepten, Dimethylsulfid, 4-Heptanon, 2(5H)-Furanon und 3-Heptanol.

5. Verfahren zum Nachweis einer Pilzkontamination gemäß einem der Ansprüche 1 bis 4, **dadurch gekennzeichnet, dass** in Schritt c) jeder der vorgegebenen VOC eine Zahl aus -1, 0 oder 1 zugeordnet wird in Abhängigkeit ihres Vorliegens oder ihrer Abwesenheit und die Summe dieser Zahlen berechnet wird, was den Kontaminationsindex ergibt.

6. Verfahren zum Nachweis einer Pilzkontamination gemäß Anspruch 5, **dadurch gekennzeichnet, dass** für jede der VOC mit Ausnahme von Methyl-2-ethylhexanoat die Zuweisung der Zahlen folgendermaßen erfolgt:
- das Vorliegen von VOC der Kategorie (1) ist durch die Zahl 1 gekennzeichnet und ihre Abwesenheit durch -1;
- das Vorliegen von VOC der Kategorie (2) ist **gekennzeichnet durch** die Zahl 0 und ihre Abwesenheit **durch** -1;
- das Vorliegen von VOC der Kategorie (3) ist **gekennzeichnet durch** die Zahl 1 und ihre Abwesenheit **durch** 0.

7. Verfahren zum Nachweis einer Pilzkontamination gemäß Anspruch 5 oder 6, **dadurch gekennzeichnet, dass** für Methyl-2-ethylhexanoat die Zuweisung der Zahlen folgendermaßen erfolgt:
- das Vorliegen von Methyl-2-hexanoat ist **gekennzeichnet durch** die Zahl 1;
- die Abwesenheit von Methyl-2-ethylhexanoat ist **gekennzeichnet durch** die Zahl 0, wenn eine Abwesenheit von 2-Ethylhexanol erkannt wird, und **durch** die Zahl-1, wenn ein Vorliegen von 2-Ethylhexanol erkannt wird.

## Claims

1. A method for detecting fungal contamination of indoor surroundings defined by a confined space inside a building which is not continuously aerated which comprises the following stages:
a. withdrawing a sample of volatile organic compounds (VOCs) in indoor surroundings,
b. detecting the presence or the absence of certain predetermined VOCs resulting from fungal metabolism, these predetermined VOCs comprising at least one VOC of each of the three following categories of VOCs:
(1) the VOCs which are given off independently of the fungal species and of its substrate and which are given off only by fungal species;
(2) the VOCs which are given off independently of the fungal species and of the substrate but which can also have other biological origins;
(3) the VOCs which are given off according to the fungal species and/or substrate;
c. calculating a chemical index for fungal contamination according respectively to the presence and absence of the predefined VOCs resulting from fungal metabolism, taking into account that:
the presence of VOCs of category (1) directly indicates the presence of fungal contamination,
while the absence of such VOCs indicates the absence of fungal contamination;
the presence of VOCs of category (2) does not allow it to be concluded that fungal contamination is present while the absence of such VOCs indicates the absence of fungal contamination; and
the presence of VOCs of category (3) indicates the presence of fungal contamination while their absence does not allow it to be concluded that fungal contamination is absent.

2. The method for detecting fungal contamination as claimed in claim 1, **characterized in that** the presence or the absence of several VOCs of each of the three VOC categories is detected.

3. The method for detecting fungal contamination as claimed in claim 1 or 2, **characterized in that** the predetermined VOCs also comprise 2-ethylhexanol, in addition to the VOCs of categories (1), (2) and (3).

4. The method for detecting fungal contamination as claimed in any one of claims 1 to 3, **characterized in that**
- the VOCs of category (1) are chosen from the group comprising 1-octen-3-ol, 1,3-octadiene and methyl-2-ethylhexanoate,
- the VOCs of category (2) are chosen from the group comprising 2-methylfuran, 3-methylfuran, 3-methyl-l-butanol, 2-methyl-1-butanol and α-pinene,
- the VOCs of category (3) are chosen from the group comprising 2-heptene, dimethyl sulfide, 4-heptanone, 2(5H)-furanone and 3-heptanol.

5. The method for detecting fungal contamination as claimed in any one of claims 1 to 4, **characterized in that**, in stage c), a figure of -1, 0 or 1 is assigned to each of the predetermined VOCs, according to its presence or its absence, and the sum of these figures, which is the contamination index, is calculated.

6. The method for detecting fungal contamination as claimed in claim 5, **characterized in that**, for each of the VOCs, with the exception of methyl-2-ethylhexanoate, the figures are assigned in the following way:
- the presence of VOC of category (1) is **characterized by** the figure 1 and its absence by -1;
- the presence of VOC of category (2) is **characterized by** the figure 0 and its absence by -1;
- the presence of VOC of category (3) is **characterized by** the figure 1 and its absence by 0.

7. The method for detecting fungal contamination as claimed in claim 5 or 6, **characterized in that**, for methyl-2-ethylhexanoate, the figures are assigned in the following way:
- the presence of methyl-2-ethylhexanoate is **characterized by** the figure 1;
- the absence of methyl-2-ethylhexanoate is **characterized by** the figure 0, if the absence of 2-ethylhexanol is detected, and by the figure -1, if the presence of 2-ethylhexanol is detected.
